# EUROPEAN PATENT APPLICATION

(11) **EP 1 857 439 A1**
(43) Date of publication of application: **21.11.2007**
(21) Application number: 06114097.6
(22) Date of filing: 17.05.2006
(51) Int. Cl.: C07C 273/16, B01D 61/02, B01D 61/08, B01D 61/14, B01D 61/18, B01D 53/94

(54) **Method for purifying aqueous urea solution**

(71) Applicant: Kemira GrowHow Oyj, 00181 Helsinki (FI)
(72) Inventor: Weckman, Anders, Espoo 02770 (FI); Lehtonen, Riku, Helsinki 00100 (FI); Palenius, Tapio, Vantaa 01350 (FI)
(74) Representative: Saijonmaa, Olli-Pekka

(57) **Abstract**

The invention relates to a method for purifying an aqueous urea solution using optionally particulate filtering and a reverse osmosis treatment. An assembly for carrying out the purification is disclosed. The purified urea solution is used in selective catalytic reduction of nitrogen oxides.

## Description

The invention relates to a method for purifying an aqueous urea solution and to an assembly for carrying out the purification. The invention relates further to the use of thus purified aqueous urea solution in catalytic reduction of nitrogen oxides.

### Background

There are about ten independent urea producers in the Western Europe whose combined capacity is about 5 million tons of solid urea per year. From this capacity about 85% is used as agricultural fertilizers and the rest is used for technical applications such as amino resins, glues and medicinal products.

In a commercial urea fertilizer production plant urea is synthesized by an exothermic reaction from liquid ammonia and gaseous carbon dioxide at high temperature and pressure to yield a mixture of urea and ammonium carbamate, which is subsequently dehydrated in an endothermic reaction to form urea and water. During the manufacture urea decomposes to some extent to form for example biuret, which is the main and least desirable by-product present in commercial urea. The design of commercial processes has involved consideration of how to separate the urea from the other constituents, how to recover excess NH3, and to decompose the carbamate for recycle. Attention has also been devoted to develop materials withstanding the corrosive carbamate solutions and to optimize the heat and energy balances. The urea solution arising from the synthesis/recycle stages of the process is subsequently concentrated into a urea melt for conversion to a solid. Due to its hygroscopic nature urea is processed further into prills or granules with the aid of quality improving agents reducing dust emissions and/or caking tendency during handling and storage. Formaldehyde in particular, is most often used alone or together with other anti-caking agents.

If a pure aqueous urea solution is required urea must be extracted from the production process plant at an intermediate stage. For example, a particularly pure method of production guaranteed to meet the high quality criteria set in e.g. the DIN standard 70070 is to extract the urea out from the urea production as a stream of very pure liquid melt. The stream of pure liquid is poured into dedicated bulk tanks where it is dissolved in pure demineralized water to produce aqueous, high quality urea solution, essentially free from impurities.

Due to the need to extract the pure urea melt from the processing stream at the manufacturing plant there arises an increased need to transport this pure aqueous urea solution for varying distances and durations before the suitable storage or actual point of use is reached. This enhances the importance of material selection for storage vessels and environmental conditioning for the storage period in order to maintain the high quality of the freshly prepared pure solution.

This type of high purity aqueous urea solution is used in e.g. selective catalytic reduction equipment (SCR) for chemically reducing nitrogen oxide emissions of heavy-duty diesel powered vehicles.

According to the European legislation (Euro IV 10/2006) commercial vehicles are only allowed to emit a certain limit of noxious fumes. To meet this legislation the amount of nitrogen oxides emitted from the truck, bus or coach exhaust cannot exceed 2 grams per kilowatt-hour. While Euro IV is to be fully implemented in October 2006, any newly designed commercial vehicle engines developed by the manufacturer after October 2006 must meet Euro IV. One way of suppressing these emissions is to use an aqueous urea solution to convert the nitrogen oxides into nitrogen and water by spraying it into the exhaust gas flow where it is subsequently hydrolyzed forming ammonia

(NH₂)₂CO + H₂O -> 2 NH₃ + CO₂

before the catalytic converter effecting the breakdown of nitrogen oxides into nitrogen and water

4 NH₃ + 4 NO + O₂ -> 4 N₂ + 6 H₂O.

The current method of extracting the required pure urea from the intermediate stage of the production process is neither a very flexible nor a readily accessible way of generating urea as consumer material for everyday use at numerous geographical locations. A special effort needs to be directed to transportation and storage facilities, materials and conditions.

Another alternative would be to dissolve purified urea into purified water at or near the point-of-use. The presently used urea purification methods for regular urea grades combined with water purification methods producing ultra pure water do not result in economically or logistically feasible solutions.

The most important and commonly known way of purifying urea is by recrystallization. The result obtained is of high quality, but the process is laborious and inefficient to implement. It requires several unit operations, such as heating, cooling, filtering, centrifuging, drying, etc. adding up to a crystallizing plant. Further more, occasionally urea tends to form complex compounds with metal ions or adducts. Frequently, urea crystallites include impurities either in the crystal lattice, adsorbed on the surface or as separate co-precipitates. Other possible, but less feasible or practical purification methods comprise for example filtering, extraction or ion exchange.

Reverse osmosis is a fairly mature technology mainly used in desalination of sea water and/or brackish water. Separation technology based on pressure driven reverse osmosis processes has been developed somewhat later. There have been considerable efforts directed to development of these methods. Another target area has been, for example, in removing urea from aqueous solutions, especially from body fluids. The preparation of suitable membranes has been a major challenge, especially in view of tolerance for different chemicals and more reasonable water fluxes. A common view in chemistry is that reverse osmosis membrane has tiny pores, less than a billionth of a meter in size, which are small enough so that water can filter through but contaminants such as urea and ammonia cannot. Similarly, in biology it is considered that biological membranes form the cell walls, and play a major role in regulating the internal environment of all living cells. They do so by allowing water to pass freely on either side of the membrane's pore, but excluding other molecules, such as urea and inorganic salts.

However, further research in the field of reverse osmosis has indicated that urea might have a different mechanism through the membranes compared to e.g. salts. Thus, depending on the membrane selection some urea may permeate which has been considered a problem. Typically, values of urea rejection such as 45% for cellulose acetate membranes and 66% for cellulose triacetate has been published the aim being increasing the rejection value of urea to as high as possible as disclosed in US4392960.

JP2000281638 discloses separation of urea as an undesired component from very dilute natural substance solutions containing minor amounts, 0.01-0.5 w-% of urea. The method includes several filtration, reverse osmosis, ion exchange and/or electrodialysis treatments for purifying these dilute solutions of e.g. skim milk or soybean protein containing urea as a contaminant or impurity.

### Object of the invention

The object of the present invention is to provide a novel method for purifying an aqueous urea solution containing undesired impurities. Another object is to purify a concentrated aqueous urea solution while essentially maintaining the initial urea concentration.

A further object of the present invention is to provide a simple and practical method to prepare a high quality aqueous urea solution that can be used for removal of nitrogen oxides from exhaust gases of diesel vehicles.

Yet, a further object of the present invention is to provide an economical, energy-saving, readily transferable assembly for the preparation of high quality aqueous urea solution.

### Description of the invention

The above-mentioned objects of the invention have now been achieved by a novel way of purifying an aqueous urea solution by reverse osmosis treatment. Surprisingly, it was found that urea even from a concentrated aqueous urea solution is able to penetrate a suitably chosen pressure driven reverse osmosis membrane with a high throughput leaving other salt-like, unwanted impurities into the feed solution. This observation enabled to selectively purify urea solutions with even high original urea content and to maintain the high urea content in the product solution.

According to the invention, there is provided a method for purification of aqueous urea solution containing at least 10 % by weight of urea by applying the following steps:
Firstly, if particulate impurities are included in the feed solution and they are considered detrimental they are essentially filtered off from the feed solution using micro filtration.
Secondly, this filtered solution from the first step is subjected to a reverse osmosis treatment resulting in a permeate solution and a reject solution.
Thirdly, the permeate solution from the second step is collected as the purified urea product solution.

This purified urea product solution obtained by applying the reverse osmosis treatment is pure enough to be used in a catalytic reduction process. Thus obtained aqueous urea solution satisfies the requirements set forth for impurities that might have a detrimental effect on the catalyst used in these processes.

The method according to the invention is carried out in an assembly comprising inlet feed means for introducing the aqueous urea containing feed solution to be purified into said assembly, attached to a micro filtration unit for removal of particulate impurities from the feed solution, connected to a reverse osmosis unit for carrying out the final purification of aqueous urea solution and finally connected to outlet feed means for ejection of the purified aqueous urea containing product solution.

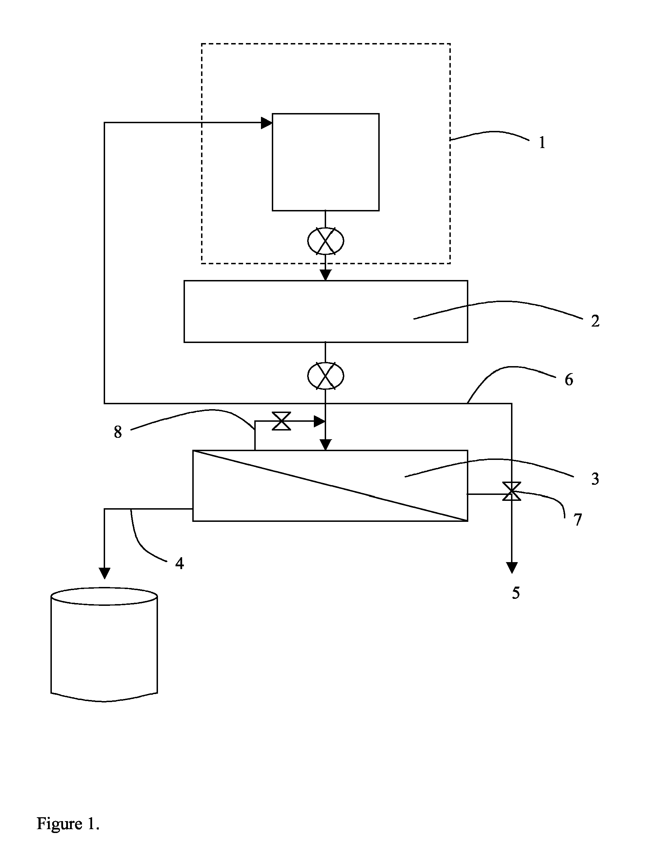

Figure 1 depicts a flow chart for one preferred assembly and method alternative for purifying an aqueous urea solution according to the method of the invention.

### Detailed description of the invention

Urea used for the preparation of the feed solution may originate from any industrial process producing urea in solid or aqueous solution form. This includes various urea grades typically used in agriculture or other applications such as fertilizer grade urea, typically including maximum of 1.2 w-% biuret and formaldehyde stabilized; foliar grade urea, typically including maximum of 0.3 w-% biuret, 2-2.5 mm granule or 1.5-2 mm prill and stabilized with formaldehyde; cattle feed grade urea, typically excluding additional substances comprising 0.5 mm prills; technical grade urea typically used for resins or melamine; urea used in pharmaceutical industry, fermenting, brewing industry and petroleum industry. The manufacturing process affects the quality and the impurity level of the urea product, thus the amounts of impurities such as biuret and formaldehyde may vary. In order to use urea according to the invention it needs not to be free from these typical impurities or contaminants. It may contain traces of biuret, aldehydes or other anticaking agents, sulphur or its compounds, chloride, nitrate or other impurities alike. Preferably, the urea grade used according to the invention is free from any coating agent and most preferably technical grade urea.

Urea must be in a solution form in order to carry out a filtration or reverse osmosis treatment. Preferably, an aqueous solution containing at least 10 % by weight of urea is used as feed solution for the process according to the invention. Most preferably, an aqueous solution containing from 10% to 50% by weight the upper limit due to the crystallization point of the liquid at the temperature used. Most preferable, from 25% to 40% by weight urea solution is used due to only a minor need, if any, for adjustment of concentration, if the product is used for e.g. selective catalytic reduction of nitrogen oxides in heavy duty diesel vehicles.

These solutions may be prepared by dissolving various grades of solid urea as described above into water. One essential advantage of the present invention is that water used for dissolving may include some impurities, as those impurities will be removed during subjecting the solution to the method of the invention. Preferably, the amounts of impurities are less than 10 times higher than the required product specification in order not to essentially affect the quality of the urea solution. Most preferably, the water used is demineralized or ion-exchanged, ultra pure or fulfills the specifications for pure water as described in, for example, Seachem Laboratories Inc., Owners manual. Preferably, the temperature of the water to be used as dissolvent is about 50°C , more preferably more than 40°C. At dissolution water or urea solution may be moderately preheated or heated for better dissolving. For a 10-40% solution up to ½ h dissolving time is required.

The aqueous urea containing feed solution may comprise soluble impurities or impurities not removable by micro filtering due to origin of the urea used, such as biuret, preferably less than 1.2 w-%; aldehydes, preferably less than 150 mg/kg; PO₄, preferably less than 15 mg/kg; Ca, preferably less than 150 mg/kg; Fe, preferably less than 3 mg/kg; Cu, Zn, Cr, Ni and Al, preferably less than 1.2 mg/kg; Mg, preferably less than 150 mg/kg and Na and K, preferably less than 15 mg/kg calculated against 100% urea. This feed solution impurity level still enables to obtain high purity product solution with a yield such as about 90%. Even 10 times higher impurity levels are tolerable, but require further purification cycles and lead to lower yields.

In a preferred embodiment, the aqueous urea containing feed solution with urea content at the upper limit such as 32.5%, results in readily usable product solution for e.g. selective catalytic reduction with only a minor decrease in concentration due to purification, the throughput being about 95%. Thus, the purification method according to the invention may advantageously be used without any further additional need for concentrating the product solution.

The first filtering step according to the invention for removal of particulate impurities from the feed solution by micro filtration is carried out using any known conventional filter or filter cartridge or any suitable single or multiple filter assembly. The main requirements for the selection of a suitable filter assembly are removal of particles from 0.1 to 1 µm and inertness towards to used solutions. The aim is to reduce the amount of disturbing insoluble particles below 20 mg/kg which has been agreed as stated in DIN 70070 as a limit suitable for pure urea solutions to be used in e.g. nitrogen oxide removal from exhaust gases of vehicles. If the amount of particles increases, for example, in the catalytic reduction applications they may clog the atomizers, initiate corrosion and cause deposits on the catalyst. There are several commercial suppliers for suitable filter assemblies.

In a preferred embodiment of the invention a filter cartridge removing 90% of particles larger than 1 µm has been found adequate to fulfill this criteria. Preferably, the filter is of polypropylene, and most preferably polypropylene filter withstanding adequately higher temperatures and pressures such as 70°C and 8.6 bar.

In another embodiment of the invention, the aqueous urea containing feed solution does not initially comprise disturbing insoluble impurities. These impurities could be either minor enough or just not possessing a disturbing character regards to the application of the product solution. In this case, naturally, the first filtering step would be in vain and is therefore by-passed. This possibility to reduce the number of method steps simplifies the assembly needed to carry out the purification, respectively.

In the second step according to the invention the essentially insoluble particle free solution from the first step is subjected to a reverse osmosis treatment. For reverse osmosis there is equipment commercially available from several suppliers provided with suitable chemical and mechanical material properties for handling urea solution such as stainless steel 304 and 316, highly alloyed austenitic Cr-Ni-steels and Cr-Ni-Mo-steels according to DIN EN 10088-1, -2 and -3 worked according to industrial standard, titanium, hastelloy c/c 276. This treatment and equipment is conventionally used in water purification or other industrial applications such as purification of process waste streams in food and beverage industry, electronic industry, glass and mirror industry, cooling water purification or wetting water applications.

The most essential component in the reverse osmosis apparatus is the membrane system. Conventionally, pure water permeates through the membrane whereas impurities such as salts, organic agents, colloidal agents and bacteria are not able to penetrate. An overpressure is typically needed for transferring pure water through the membrane into the more dilute solution (reverse osmosis). In case of the invention urea dissolved in water is forced through the membrane together with pure water using overpressure. Preferably, an operating pressure from 1 bar to 100 bar is used, more preferably from 10 bar to 50 bar, which is a suitable pressure range for most of the commercial membrane types, most preferably from 20 bar to 45 bar. The maximum pressure difference over the membrane is typically about 1 bar. The required overpressure depends on, for example, the concentration differences at the two sides of the membrane, desired capacity and the operating temperature.

According to the invention suitable membrane materials to be used should be chemically compatible with aqueous urea solutions and mechanically durable enough for the operating conditions in question. At least membranes such as cellulose acetate, cellulose triacetate, linear and cross-linked aromatic polyamides and alkyl-alkyl polyether urea are usable. Several different types of suitable membranes are commercially available from several suppliers, thus the list presented is not exhaustive. A man skilled in the art is capable of selecting a suitable membrane material for the purpose of the present invention.

In one embodiment of the invention a membrane for clean water designed for low salinity and a pressure less than 21 bar is used.

In another embodiment of the invention a membrane for brackish water designed for higher salinity and maximum pressure of 41 bar, pH 2-11, 45°C maximum operating temperature, such as XLE-4040 from Dow Chemical Company or alike, is used.

The method according to the invention may comprise at least one recycling step d, wherein the reject solution and/or permeate solution is recycled back to the process. The amount of reject solution recycled is at maximum from 10 w-% to 90 w-%. A preferable range of usage is from 25 w-% to 75 w-%. The best quality for the product solution is obtained by using most preferably from 50 w-% to 75 w-%, which differs essentially from that used for water purification, 25-50 w-%. In general, the amount of reject solution to be circulated depends on the desired yield.

A preferred embodiment of the invention is further described by figure 1. The inlet feed means 1 comprising a storage container or a preparation unit for dissolving urea into water for the aqueous urea containing feed solution and a membrane pump, is used for transferring the feed solution to the micro filtration unit 2 filter cartridge for removal of insoluble particulate impurities. After micro filtering, the filtered aqueous urea containing solution is introduced into the membrane cartridge of the reverse osmosis treatment unit 3 via a high-pressure pump. The solution to be purified is divided into permeate 4 and reject 5, 6 streams at the membrane, the permeate solution comprising essentially of purified, dissolved urea and water, and the reject solution comprising essentially of the impurities, urea and water. The permeate is recovered as purified product solution. Part of the reject solution is recycled back to the process 6 and the rest is removed from the process as waste 5. The ratio of the recycled reject solution and removed reject solution is regulated by valve 7.

Part of the reject solution may be internally recycled in the equipment by controlling with a valve 8. The main purpose is to increase flow rate parallel to the membrane and to reduce clogging tendency. The amount of reject solution leaving the equipment is adjusted valve 7. Part of this reject stream can be returned to the feed liquid entering the equipment in order to increase the product yield.

The yield of the method depends on the throughput of the reverse osmosis treatment and the quality requirements. Throughput is determined by, for example, the volumes and ratios of the feed solution and product and reject waste solutions, pressures used for the particulate filtering step and reverse osmosis treatment, solution flow rate and type at the reverse osmosis membrane, impurity content of the feeding solution, and temperature of the feed solution.

The fouling tendency of the membranes may be decreased by applying a pulsation to the feed stream or by other means, which create turbulence or increased flow rate.

The typical capacity when purifying concentrated urea solutions is ca. 10-15 % of that for pure water.

In a preferred embodiment the once through yield is about 67%.

Depending on how much reject is returned back to the process the yield may be increased up to about 90 %. The reject can be recycled as long as the product quality requirements are fulfilled.

Because the product quality is about 10-fold compared to the reject for many ions, the yield e.g. for a feed with a 4, 2 or 1,5 times higher impurity level than the product requirement would be 70, 90 and 95 %, respectively. For purification of technical grade urea with impurity levels only slightly exceeding the requirements the yield is thus 85-95 %.

Temperature of the feed solution is less than 45°C, preferably from 0°C to 45°C due to risk for crystallization or freezing lower temperature and gradual decomposition of urea at elevated temperature, more preferably from 10°C to 40°C, most preferably from 15°C to 30°C to avoid need for cooling or heating the liquids.

The method according to the invention may comprise an additional final step for adjusting the urea content of the product solution. The urea concentration adjustment may be realized by, for example, adding solid urea or more concentrated urea solution to the feed solution or by diluting or concentrating the feed solution by any commonly known method readily available for the man skilled in the art. Alternatively, the urea content may be adjusted after the purification treatment by e.g. dissolving additional pure urea into the product solution. These adjustments to the urea content will naturally affect the product solution density and N-content.

The purification according to the method of the invention is carried out either in once through mode or by continuously recycling the reject solution. The most efficient purification is realized by removal of the reject at the expense of the yield. Whereas increasing the recycling the yield may be increased. A man skilled in the art is able to find the optimized procedure for his needs in terms of purity and yield.

The product solution directly obtainable by the method according to the invention is of high purity and may be used for example in catalytic reduction processes for reduction of nitrogen oxides in heavy duty vehicles such as diesel vehicles, ships, power plants, fuel boiler applications. The product solution fulfills the following quality specifications determined by test methods (DIN 70071 or ISO/PAS 22241-2) and is according to the German standard DIN 70070 and/or ISO/PAS 22241-1:

| | |
|---|---|
| urea, w-% | 31.8-33.2 |
| density at 20 °C, kg/m³ | 1087.0-1092.0 |
| refractive index at 20 °C | 1.3814-1.3843 |
| alkalinity as NH3, % | max. 0.2 |
| biuret, w-% | max. 0.3 |
| insolubles, mg/kg | max. 20 |
| aldehydes, mg/kg | max. 5 |
| PO4, mg/kg | max. 0.5 |
| Ca, mg/kg | max. 0.5 |
| Fe, mg/kg | max. 0.5 |
| Cu, mg/kg | max. 0.2 |
| Zn, mg/kg | max. 0.2 |
| Cr, mg/kg | max. 0.2 |
| Ni, mg/kg | max. 0.2 |
| Al, mg/kg | max. 0.5 |
| Mg, mg/kg | max. 0.5 |
| Na, mg/kg | max. 0.5 |
| K, mg/kg | max. 0.5 |

The product solution fulfilling the above described specifications may be produced by the inventive method described herein at any suitable location from the point of urea production facility to the point of end-use. The method according to the invention may be carried out in a movable assembly.

This assembly comprises inlet feed means for introducing the aqueous urea containing feed solution to be purified into said assembly. This inlet means may include a unit for preparation of the feed solution from solid urea and water or a ready to use feed solution storage. Additionally, the inlet means includes pumping means. Typically, the feed solution is introduced into the first micro filtration unit using a pump suitable for handling urea solution, valves regulating the mass flow etc. Preferably, the installation is adapted to withstand conditions such as pressure up to 5 bar and temperature up to 45°C.

The inlet feed is optionally attached to the micro filtration unit for removal of particulate impurities from the feed solution if necessary. This unit is commercially available.

The micro filtration unit is connected to a commercial reverse osmosis unit for carrying out the final purification of aqueous urea solution. Typically these commercial units include internal prefiltering system, which may be by-passed.

The reverse osmosis unit is further connected to outlet feed means for ejection of the purified aqueous urea containing product solution. Preferably, the assembly includes common means for carrying out the reject solution recycling. In addition, the outlet feed means may comprise a concentrating unit for carrying out step e.

The assembly dimensions and construction using commercially available parts enable an easy assembly, which is even transferable from one location to another with moderate effort. Furthermore, a separate source for purified water is preferably not required.

One aspect of the invention is to use the product solution obtainable by the method in reducing nitrogen oxide emissions. For the functionality of the catalyst used in the selective catalytic reduction it is essential that when injected into the hot exhaust gas the urea solution does not contain any detrimental contaminating agents. Besides this application, pure urea solution may be used in several other applications such as in irrigation fertilization or deicing at airports or roads or other applications like these where solution is sprayed through nozzles or otherwise particulate free and/or pure urea solution is required.

The method of the invention is a simple way to produce high quality, low impurity content aqueous urea solutions. Especially, impure starting material for urea and water may be utilized for the process increasing the flexibility. The method is readily available for persons of average skills as the parts of the assembly to be used to realize the method steps are available on the market by several suppliers. The assembly need not to be a large one and thus does not require large production space. Using the method according to the invention does neither require heavy investments on technical apparatus nor facilities. As the method is not based on phase changes it requires minimal amount of energy. Even post concentrating may be omitted by choosing higher original feed solution concentrations. In case the need for urea solution purification is only minor or temporary, this versatile apparatus is easily modified into water treatment. On the other hand, the capacity is easily scaled up.

The invention is described further by the following examples but not to be restricted thereto.

### Example 1

The urea yield of a urea solution subjected to purification according to the invention was tested.

A urea containing feed solution was prepared by dissolving 100 kg of commercially available technical grade urea in ion-exchanged water (grade 3 of SFS-ISO 3696). A spun polypropylene filter cartridge (P1-20, HOH Separtec Oy) was used at 20°C for removing particulate impurities from the aqueous urea containing feed solution. The filtered solution was fed into a reverse osmosis equipment (Eurotec 01-1 from Hyxo) utilizing a polyamide thin membrane filter XLE-4040 (Dow Chemical Company) designed for clean water of low salinity and less than 21 bar pressure bypassing the included cartridge filter by the manufacturer. The flow rate of the feed solution from a 1 m³ plastic container was adjusted to 37,1 kg/h product solution with a membrane pump.

The other valve was used for a small (about 10 % of the flow-through capacity) internal recycling and the other for adjusting the reject amount into 62.5% of the total feed of 99 kg/h. The pressure at the inlet feed to the membrane was about 13-19 bar and the temperature about 25-35°C due to warming of the circulated material. The product capacity was about 32-35 kg/h.

The analysis of the feed solution before and after purification is shown in table 1.

**Table 1.**

| | Feed solution | Purified product solution |
|---|---|---|
| Density (20oC), g/cm3 | 1.087 | 1.084 |
| N-tot, % | 15.0 | 14.4 |
| urea, w-% | 31.9 | 30.7 |
| biuret, w-% | 0.25 | 0.18 |
| insolubles, mg/kg | - | - |
| aldehydes, mg/kg | 1.5 | 0.2 |
| P04, mg/kg | <0.5 | <0.5 |
| Ca, mg/kg | 0.2 | 0.2 |
| Fe, mg/kg | 0.1 | <0.1 |
| Cu, mg/kg | <0.05 | <0.05 |
| Zn, mg/kg | <0.05 | <0.05 |
| Cr, mg/kg | 0.08 | <0.05 |
| Ni, mg/kg | 0.07 | <0.05 |
| Al, mg/kg | <0.1 | <0.1 |
| Mg, mg/kg | <0.1 | <0.1 |
| Na, mg/kg | <0.1 | <0.1 |
| K, mg/kg | 0.5 | <0.1 |

The urea content of the purified product solution after the purification treatment was only slightly decreased from the feed value of 31,9 w-% to 30,7 w-% which still gives above 96% throughput for the purification method.

### Example 2

The purification capacity of the method towards several salts was tested according to the invention. The same particulate filter and reverse osmosis set-up was used as in example 1.

A urea containing feed solution was prepared by dissolving 100 kg of technical grade urea in ion exchanged water and adding 20 mg/kg of PO₄ (monopotassium phosphate), 20 mg/kg of Na (NaCl), 20 mg/kg of K (KCl), 10 mg/kg of Ca (CaCl₂), 10 mg/kg of Mg (MgSO₄*7H₂O) and 10 mg/kg of Fe (Fe(SO₄)₃). However, a precipitation of Fe formed after a few hours from the preparation of the feed solution and the precipitation was removed by filtration.

The other valve was used for a small internal recycling and the other for adjusting the reject amount into 73.8 w-% of the total feed of 96 kg/h.

Table 1 shows the Analysis of the feed solution and the product solution after purification.

**Table 2.**

| | Feed solution | Purified product solution |
|---|---|---|
| Density (20°C), g/cm³ | 1.087 | 1.083 |
| N-tot, % | 14.9 | 14.3 |
| urea, w-% | 31.7 | 30.5 |
| biuret, w-% | 0.24 | 0.15 |
| insolubles, mg/kg | - | - |
| aldehydes, mg/kg | 1.8 | 0.2 |
| PO_{4,} mg/kg | 5.8 | <0.5 |
| Ca, mg/kg | 7 | <0.1 |
| Fe, mg/kg | 0.4 | 0.2 |
| Cu, mg/kg | <0.05 | <0.05 |
| Zn, mg/kg | <0.05 | <0.05 |
| Cr, mg/kg | <0.05 | <0.05 |
| Ni, mg/kg | <0.05 | <0.05 |
| Al, mg/kg | <0.1 | <0.1 |
| Mg, mg/kg | 9 | <0.1 |
| Na, mg/kg | 18 | 3 |
| K, mg/kg | 20 | 3 |

From the results in table 2 it is evident that purification takes place for organic compounds (biuret and aldehydes), anions (phosphate), the major cations (Ca, Mg, K and Na) and even for the minor cations such as Fe. In this experiment some of the cations such as Cu, Cr, Ni and Al were below the detection limits for in feed solutions.

### Example 3

The purification capacity of the method towards fertilizer quality urea as starting material was tested. The same particulate filter and reverse osmosis set-up was used as in example 1.

A urea containg feed solution was prepared by dissolving 100 kg of commercially available fertilizer grade urea coated with a typical formaldehyde method in ion exchanged water

The other valve was used for a small internal recycling and the other for adjusting the reject amount into 59.7 w-% of the total feed of 69.5 kg/h.

Table 3 shows the analysis of the feed solution and the product solution after purification.

**Table 3.**

| | Feed solution | Purified product solution |
|---|---|---|
| Density (20°C), g/cm³ | 1.088 | 1.084 |
| N-tot, % | 14.9 | 14.4 |
| urea, w-% | 31.7 | 30.7 |
| biuret,w- % | 0.28 | 0.21 |
| insolubles, mg/kg | - | - |
| aldehydes, mg/kg | 315 | 70 |
| PO4, mg/kg | 0.7 | <0.5 |
| Ca, mg/kg | 0.2 | <0.1 |
| Fe, mg/kg | 0.1 | <0.1 |
| Cu, mg/kg | <0.05 | <0.05 |
| Zn, mg/kg | <0.05 | <0.05 |
| Cr, mg/kg | <0.05 | <0.05 |
| Ni, mg/kg | <0.05 | <0.05 |
| Al, mg/kg | 0.1 | <0.1 |
| Mg, mg/kg | 0.1 | <0.1 |
| Na, mg/kg | 2 | 0.1 |
| K, mg/kg | <0.1 | <0.1 |

The analysis results from table 3 show that purification the highly elevated level of aldehydes in the feeding solution can be considerable decreased. Simultaneously, there is a slight decrease in both the anion (PO₄) and the major cation (Ca, Na) contents.

### Example 4

The purification capacity was tested. The same particulate filter and reverse osmosis set-up was used as in example 1.

The urea content and related density of a urea product solution was adjusted by increasing the concentration of the feed solution to the reverse osmosis. Additional urea of 2.7 w-% was added to the feeding solution of examples 1 and 2, and 3,0 w-% to example 3.

With these adjustments the urea contents of product solutions from examples 1, 2 and 3 comprise 32,5 w-% and densities of 1,087 (g/cm³, 20 °C) was reached as shown in table 4. The small concentration change did not affect the other analyses results.

**Table 4.**

| | Adjusted from example 1 | Adjusted from example 2 | Adjusted from example 3 |
|---|---|---|---|
| Density (20°C), g/cm³ | 1.087 | 1.087 | 1.087 |
| N-tot, % | | | |
| urea, w-% | 32.5 | 32.5 | 32.5 |
| biuret,w- % | 0.18 | 0.21 | 0.15 |
| insolubles, mg/kg | nd | nd | nd |
| aldehydes, mg/kg | 0.2 | 70 | 0.2 |
| PO₄, mg/kg | <0.5 | <0.5 | <0.5 |
| Ca, mg/kg | 0.2 | <0.1 | <0.1 |
| Fe, mg/kg | <0.1 | <0.1 | 0.2 |
| Cu, mg/kg | <0.05 | <0.05 | <0.05 |
| Zn, mg/kg | <0.05 | <0.05 | <0.05 |
| Cr, mg/kg | <0.05 | <0.05 | <0.05 |
| Ni, mg/kg | <0.05 | <0.05 | <0.05 |
| Al, mg/kg | <0.1 | <0.1 | <0.1 |
| Mg, mg/kg | <0.1 | <0.1 | <0.1 |
| Na, mg/kg | <0.1 | 0.1 | 3 |
| K, mg/kg | <0.1 1 | <0.1 | 3 |
| | | | |

| | | | |
|---|---|---|---|
| nd= not detected | | | |

Alternatively, equal adjustment was made after the purification treatment by dissolving pure urea into the product solution and the same results were obtained.

### Example 5

The effect of the reject amount was tested with four urea feed solutions by measuring the electric conductivity (EC) of the reject and product stream. As can be seen from the table 5 the ratio between EC in reject to product stays relatively constant throughout the whole range. This means that the permeation of salts through the membrane is related to the concentration .

The quality of the product improves when a higher reject split is used as can be seem from table 6. The initial feed solution is marked as 0 % reject in the table.

**Table 5.**

| % reject | EC reject/ EC feed | | | |
|---|---|---|---|---|
| | Feed No.1 | Feed No.2 | Feed No.3 | Feed No.4 |
| 23.2 | 2.31 | | | |
| 62.5 | 2.36 | | | |
| 91.3 | | 2.31 | | |
| 59.7 | | 2.53 | | |
| 50.8 | | 2.54 | | |
| 34.5 | | 2.69 | | |
| 2 | | 2.3 | | |
| 42.5 | | | 3.58 | |
| 0.1 | | | 2.84 | |
| 0.1 | | | 2.56 | |
| 89.2 | | | | 5.12 |
| 76.8 | | | | 4.95 |
| 60.8 | | | | 4.81 |
| 32.4 | | | | 4.48 |
| 17.2 | | | | 4.46 |

**Table 6.**

| % reject | EC, µS/cm product | | | |
|---|---|---|---|---|
| | Feed No.1 | Feed No.2 | Feed No.3 | Feed No.4 |
| 62.5 | 187.4 | | | |
| 23.2 | 231 | | | |
| 0 | 325 | | | |
| 91.3 | | 142.2 | | |
| 59.7 | | 147.9 | | |
| 50.8 | | 161.1 | | |
| 34.5 | | 181 | | |
| 2 | | 260 | | |
| 0 | | 272 | | |
| 42.5 | | | 260 | |
| 0.1 | | | 387 | |
| 0.1 | | | 429 | |
| 0 | | | 602 | |
| 89.2 | | | | 119.1 |
| 76.8 | | | | 144.2 |
| 60.8 | | | | 166.4 |
| 32.4 | | | | 283 |
| 17.2 | | | | 316 |
| 0 | | | | 559 |

## Claims

**1.** A method for the purification of aqueous urea solution **characterized in that** a feed solution containing at least 10 % by weight of urea is purified by the following steps:
a. optionally particulate impurities included in the feed solution are filtered off from said solution by micro filtration,
b. the feed solution or the filtered solution is subjected to a reverse osmosis treatment resulting in a permeate solution and a reject solution, and
c. the permeate solution is collected as the purified urea product solution.

**2.** A method according to Claim 1, **characterized in that** the method comprises at least one recycling step d whereby the reject solution and/or permeate solution from step b is recycled back to step a or b.

**3.** A method according to Claim 2, **characterized in that** the amount of reject solution recycled is from 10% to 90%, preferably from 25% to 75%, most preferably from 50% to 75% by weight.

**4.** A method according to Claim 1, **characterized in that** the feed solution of step a is an aqueous urea solution containing impurities.

**5.** A method according to Claim 4, **characterized in that** the impurities in the aqueous urea solution comprise biuret, aldehydes, insolubles, phosphate anions and/or cations such as Ca, Fe, Cu, Zn, Cr, Ni, Al, Mg, Na and/or K.

**6.** A method according to Claim 5, **characterized in that** the amount of impurities are for biuret is less than 1.2% by weight, aldehydes less than 150 mg/kg, phosphate anions less than 15 mg/kg, Ca less than 150 mg/kg, Fe less than 3 mg/kg, Cu, Zn, Cr, Ni and Al less than 1.2 mg/kg, Mg less than 150 mg/kg and Na and K less than 15 mg/kg calculated against 100% urea.

**7.** A method according to Claim 1, **characterized in that** the feed solution in step a is prepared by dissolving urea containing impurities into water.

**8.** A method according to Claim 7, **characterized in that** urea used in preparing the feed solution of step a originates from fertilizer grade urea, foliar grade urea, cattle feed urea, technical grade urea or other industrial grade urea.

**9.** A method according to Claim 7, **characterized in that** the water used for dissolving urea contains impurities.

**10.** A method according to Claim 7, **characterized in that** the water is essentially pure such as demineralized or ion -exchanged water.

**11.** A method according to Claim 1, **characterized in that** the micro filtration in step a is carried out using a filter removing at least 90% of particles larger than 1 µm.

**12.** A method according to Claim 1, **characterized in that** the particulate impurities filtered off at step a are from 0.1 µm to 1 µm.

**13.** A method according to Claim 1, **characterized in that** the micro filtration in step a is carried out using a conventional filter, filter cartridge or a multiple filter assembly.

**14.** A method according to Claim 1, **characterized in that** the reverse osmosis treatment of step b is carried out using a pressure from 1 bar to 100 bar, preferably from 10 bar to 50 bar, more preferably from 20 bar to 45 bar.

**15.** A method according to Claim 1, **characterized in that** the reverse osmosis treatment of step b is carried out using a feed solution temperature up to 45°C, preferably from 0°C to 45°C, more preferably from 10°C to 40°C, most preferably from 15°C to 30°C.

**16.** A method according to Claim 1, **characterized in that** the feed solution contains urea from 10% to 50% by weight, preferably from 25% to 40 %.

**17.** A method according to Claim 1, **characterized in that** the purified urea product solution obtained from step c contains impurities in addition to urea less than; 0.5 mg/kg Fe, 0.2 % NH3 as alkalinity, 20 mg/kg insolubles, 0.5 mg/kg phosphate anions, 0.5 mg/kg Ca, 0.5 mg/kg Mg, 0.5 mg/kg Na and 0.5 mg/kg K.

**18.** A method according to Claim 1, **characterized in that** the method comprises an additional final step for adjusting the urea content of the product solution comprising concentrating the product solution or adding solid urea or urea solution into the product solution.

**19.** Use of a purified urea product solution obtained by the method of claim 1 in a catalytic reduction process.

**20.** Use according to Claim 19, **characterized in that** the catalytic reduction process is a selective catalytic reduction of nitrogen oxides in heavy-duty vehicles such as diesel vehicles, ships, power plants or fuel boiler applications.

**21.** Use of reverse osmosis treatment for purifying aqueous urea solution comprising urea at least 10% by weight and containing impurities.

**22.** Use according to claim 21, **characterized in that** the impurities comprise biuret, aldehydes, insolubles, phosphate anions and/or cations such as Ca, Fe, Cu, Zn, Cr, Ni, Al, Mg, Na and/or K.

**23.** A method according to claim 22, **characterized in that** the amount of impurities are for biuret are less than 1.2% by weight, aldehydes less than 150 mg/kg, phosphate anions less than 15 mg/kg, Ca less than 150 mg/kg, Fe less than 3 mg/kg, Cu, Zn, Cr, Ni and Al less than 1.2 mg/kg, Mg less than 150 mg/kg and Na and K less than 15 mg/kg calculated against 100% urea.

**24.** An assembly for producing urea solution according to the method in claim 1 **characterized in that** it comprises
a. an inlet feed means for introduction of aqueous urea solution to be purified into said assembly,
b. attached to the inlet feed means, optionally, a micro filtration unit_for removing particulate impurities from the aqueous urea solution,
c. connected to the inlet feed means or to the micro filtration unit a reverse osmosis unit for carrying out the final purification of aqueous urea solution from the micro filtration unit,
d. connected to the reverse osmosis unit, an outlet feed means for ejection and storage of the purified aqueous urea containing product solution.

**25.** An assembly according to claim 24, **characterized in that** the product solution contains impurities in addition to urea less than; 0.5 mg/kg Fe, 0.2 % NH3 as alkalinity, 20 mg/kg insolubles, 0.5 mg/kg phosphate anions, 0.5 mg/kg Ca, 0.5 mg/kg Mg, 0.5 mg/kg Na and 0.5 mg/kg K.

**25.** An assembly according to claim 24, **characterized in that** the inlet feed means comprises a unit for dissolving urea into water.

**26.** An assembly according to claim 24, **characterized in that** the outlet feed means comprises a unit for adjusting the concentration of urea product solution.
